# EUROPEAN PATENT APPLICATION

(11) **EP 3 587 647 A1**
(43) Date of publication of application: **01.01.2020**
(21) Application number: 19181886.3
(22) Date of filing: 24.06.2019
(51) Int. Cl.: D04H 13/00, G01L 5/00, A61F 13/15, G01B 5/28, G01N 33/36, G01B 21/30

(54) **METHOD FOR DETECTING DEFECTS IN A NONWOVEN SUBSTRATE**

(30) Priority: 28.06.2018 EP 18180273
(71) Applicant: The Procter & Gamble Company, Cincinnati, OH 45202 (US)
(72) Inventor: FLOETER, Stefan, 53881 Euskirchen (DE); GABER, René, 53881 Euskirchen (DE); VARGA, Stephen Michael, Cincinnati, OH 45202 (US)
(74) Representative: P&G Patent Germany

(57) **Abstract**

The present invention relates to a method for detecting defects in a web (30) of nonwoven fibrous material, wherein the nonwoven fibrous material comprises natural fibres, regenerated cellulose fibres, and mixtures thereof, comprising the steps of:
advancing the nonwoven web (30) in contact with a roll through a detection zone (40);
providing a sensor (20) in the detection zone (40) which detects variations in pressure between the nonwoven web (30) and the surface of the sensor (20);
generating a signal from the sensor (20) when variations in pressure indicate the presence of a defect.

## Description

### FIELD OF THE INVENTION

The present invention relates to methods of inspecting nonwoven substrates. In particular the present invention relates to methods of detecting defects caused by particles in webs made from fibrous materials comprising natural fibres and/or regenerated cellulose fibres.

### BACKGROUND OF THE INVENTION

Disposable wipes, either wet or dry, are well-known and successfully commercialized for a large variety of uses. For instance, wipes may be used for cleaning hard surfaces such as floors or kitchen surfaces. Wipes may also be used for personal cleaning, for example to remove facial make-up or to clean or refresh the skin whilst traveling. Wipes are also particularly appreciated for cleaning baby's skin in the perineal area during a diaper change.

Typically, wipes comprise a substrate, in the form of a nonwoven sheet or web. The sheet may be impregnated with a lotion composition wetting the substrate to facilitate cleaning and providing a so-called wet wipe. The lotion composition may deliver additional benefits, e.g. soothing or treating.

Various types of substrates, differing in their visual and tactile properties, may be utilized for manufacturing disposable wipes. When wipes are intended to be used as personal care wipes, such as baby wipes, facial cleansing wipes, intimate cleansing wipes, and the like, softness, flexibility, coverage, effective cleaning ability, thickness, strength and opacity are properties that matter for the consumers. Substrates should also exhibit a homogeneous texture, free of particles or "lumps". Undesirable particles may be present as debris, dust, particles, or clumps of fibres.

Existing methods of inspecting substrates for the presence of particles include optical sensors, cameras etc. However, these systems are often unsatisfactory. Either the sensitivity of the optical system is too low to detect particles and some particles pass undetected, or the sensitivity of the optical system may be increased, but then the system generates too many false positives, rejecting product which is of good quality.

Thus, it would be desirable to accurately detect the presence of undesired particles in a web of nonwoven fibrous material comprising natural fibres or regenerated cellulose fibres so that defective product can be rejected.

### SUMMARY OF THE INVENTION

The present invention provides a method for detecting defects in a web of nonwoven fibrous material, wherein the nonwoven fibrous material comprises natural fibres, regenerated cellulose fibres, and mixtures thereof, comprising the steps of:
advancing the nonwoven web in contact with a roll through a detection zone;
providing a sensor in the detection zone which detects variations in pressure between the nonwoven web and the surface of the sensor;
generating a signal from the sensor when variations in pressure indicate the presence of a defect.

### BRIEF DESCRIPTION OF THE DRAWINGS

Figure 1 is a schematic drawing showing an embodiment of the present invention comprising two rollers.
Figure 2 is a schematic drawing showing an alternative embodiment of the present invention comprising one roller.
Figures 3 and 4 are schematic drawings showing alternative embodiments of the present invention comprising a roller and a pressure sensitive surface.

### DETAILED DESCRIPTION OF THE INVENTION

The present invention provides a method for detecting defects in a nonwoven substrate wherein the nonwoven substrate comprises fibrous material. The defects comprise areas of the nonwoven substrate in which fibrous material has fused to form particles. The defects may be categorized as clumps of fibres; thick fibres; fibers which have melted and resolidified/fused; or debris/contamination.

Fibrous materials of natural origin invariably contain particulate dirt, dust and debris which translates as a product defect if it is present in the nonwoven substrate. Such fibrous materials are cleaned in order to remove particulate dirt, dust and debris before the fibrous materials are converted into nonwoven substrates. Cleaning processes include, for example, physical steps such as combing. Achieving a pure fibrous material, completely free of particulate dirt, dust and debris, is an expensive process. It would be more economical to partly clean the fibrous material by removing only the most easily removeable particulate dirt, dust and debris, but leaving some particulate residues that would be difficult and expensive to remove completely. However this results in some product defects in the nonwoven substrate.

Regenerated cellulose fibres may form into clumps caused by fibres fusing together when still molten or soft immediately after spinning. Such clumps of fused fibres result in product defects in the nonwoven substrate.

The present invention addresses these problems by providing a tactile sensing method of detecting defects in nonwoven substrates which comprise natural fibres and/or regenerated cellulose fibres.

The term "wipe" as used herein, refers to an article comprising a sheet of fibrous material. Wipes are also known to as "cleaning sheet". Wipes, either dry or wet, are intended to be used for removal of a substance from a surface or object which is animate or inanimate, or alternatively, application of a material to a surface or object which is animate or inanimate. For instance, wipes may be used for cleaning hard surfaces, such as floors. Wipes may also be used for human or animal cleansing or wiping such as anal cleansing, perineal cleansing, genital cleansing, and face and hand cleansing. Wipes may also be used for application of substances to the body, including but not limited to application of make-up, skin conditioners, ointments, and medications. They may also be used for cleaning or grooming of pets. Additionally, they may be used for general cleansing of surfaces and objects, such as household kitchen and bathroom surfaces, eyeglasses, exercise and athletic equipment, automotive surfaces, and the like.

The wipe may have a variety of shapes, including but not limited to, circular, square, rectangular, oval, or irregularly shaped. However, generally, a wipe is rectangular or square in shape and is defined by two pairs of opposite sides or edges. Each wipe has a width and a length. For example, the wipe may have a length of from about 6 to about 40 cm, or from about 10 to about 25 cm, or from about 15 to about 23 cm, or from about 17 to about 21 cm and may have a width of from about 10 to 25 cm, or from about 15 to about 23 cm, or from about 17 to about 21 cm. Each individual wipe may be arranged in a folded configuration and stacked one on top of the other to provide a stack of wipes. Such folded configurations are well known to those skilled in the art and include c-folded, z-folded, quarter-folded configurations and so forth.

The term "denier" as used herein refers to a unit used to indicate the fineness of a filament/fiber. The unit expresses the mass of a filament/fiber in grams per 9000 meters of length.

As used herein with respect to the fibrous material, the term CD or "cross-direction" refers to the direction, in the plane of the fibrous material, perpendicular to the machine-direction. The term "machine-direction" refers to the direction of travel as the fibrous material is produced, for example on nonwoven making equipment. With respect to individual wipes or sheets, the terms "machine-direction" and "cross-machine direction" refer to the corresponding directions of the wipes/sheets with respect to the fibrous material the wipe/sheet was made from.

The term "gsm" as used herein refers herein to "grams per square meter" (g/m²).

The term "basis weight" as used herein refers to the weight per unit area of the wipe.

The term "thermoplastic" as used herein refers to a polymer that flows under shear when exposed to heat and returns to its original condition when cooled to room temperature. Examples of thermoplastic materials include, but are not limited to, styrene polymers and copolymers, acrylics, polyester, polylactic acid, cellulose acetate, polyethylenes, polypropylenes, vinyls and nylon.

In one embodiment of the present invention a tactile sensor is used to detect pressure variations which are caused by the presence of particles in the nonwoven substrate.

It is known to use pressure sensors in order to monitor roll alignment in the nip,

For example, US 5,562,027, issued on October 8th 1996, discloses rolls having sensors embedded in the roll cover. The sensors are used to measure pressure or temperature and may be configured to measure the pressure profile in the nip in order to detect misalignments of the rolls.

Suitable tactile sensors are available commercially. One tactile sensor useful for the present invention is the Pressure Mapping Sensor 5040 supplied by Tekscan, South Boston, Massachusetts, USA.

Preferably the tactile sensor is configured to measure pressure up to about 3500 kPa, preferably between about 350 kPa and about 3500 kPa.

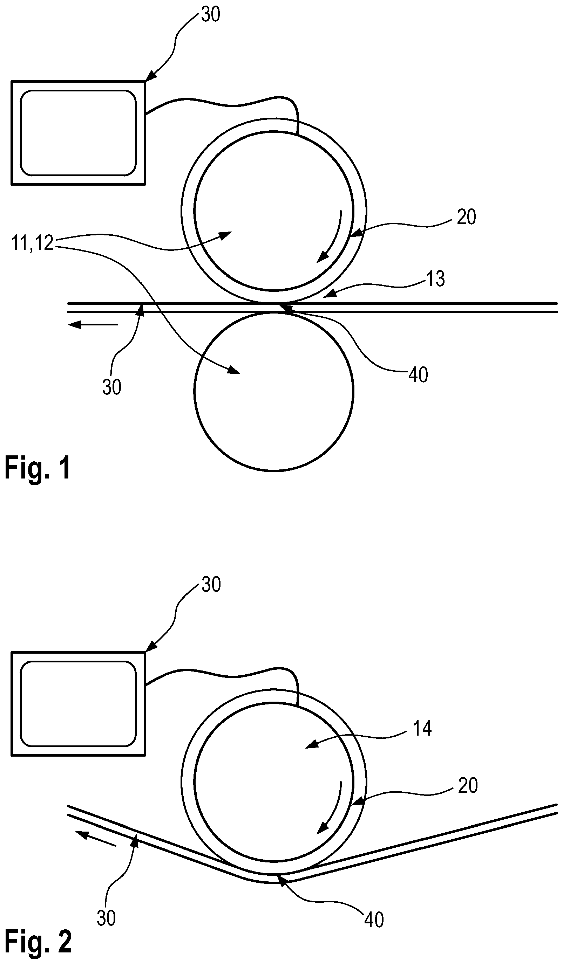

Figure 1 shows a system wherein the web 30 is transported between two opposing surfaces, preferably the two opposing surfaces are opposing surfaces of adjacent rotating rolls 11, 12. In this embodiment pressure is applied to the nonwoven substrate by transporting the nonwoven substrate through a nip 13 between the rotating rolls 11, 12. Alternatively the nonwoven substrate may be transported over slide bars, i.e. bars which do not rotate or which rotate at a slower speed relative to the speed of the web.

In an alternative embodiment of the invention, shown in Figure 2, the pressure is generated by the tension in the web 30 partially wrapped around the roller 14 in which a tactile sensor 20 is embedded.

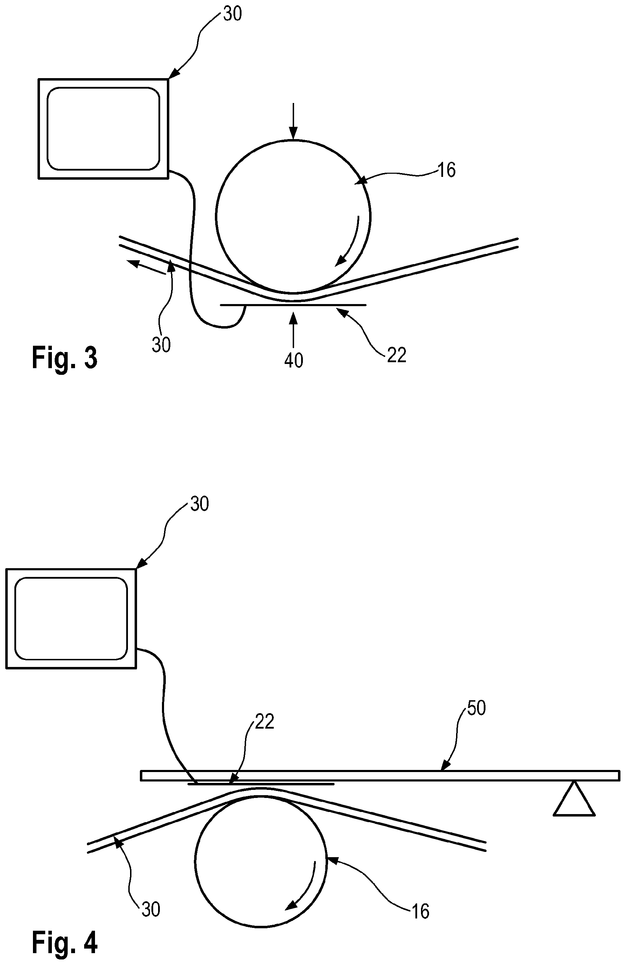

In another alternative embodiment of the invention, shown in Figure 3, the tactile sensor 22 is static while the web 30 is sliding past. In a first option the roll 16 pressing the web 30 against the tactile sensor 22 is moving and applying the desired pressure. In another alternative embodiment, shown in Figure 4, the tactile sensor 22 is mounted on a lever 50 which is pressed against the web 30 running across a roller 16.

The signal of the tactile sensor may be processed using a processor 30. For example the signal may be processed using image analysis techniques analogous with those techniques used in vision systems, for example vision systems comprising a camera. The signal of the tactile sensor may also be processed as a cross-direction inline sensor whereby the signature from the contact point is representative of a characteristic of a web component.

In another embodiment of the present invention an imprint of the profile of the pressure is created on at least one of the opposing surfaces. The imprinted surface is then inspected. For example, the imprinted surface may be inspected by an optical sensor or camera which is configured to detect imprints in the surface which have been left by a particle.

The present invention may be applied to webs of fibrous materials comprising natural fibres, regenerated cellulose fibres, or combinations thereof. Examples of natural fibres include, but are not limited to, cotton, hemp, flax. Examples of regenerated cellulose fibres include, but are not limited to, viscose, rayon, lyocell.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A method for detecting defects in a web (30) of nonwoven fibrous material, comprising the steps of:
advancing the nonwoven web (30) in contact with a roll through a detection zone (40);
providing a sensor (20) in the detection zone (40) which detects variations in pressure between the nonwoven web (30) and the surface of the sensor (20);
generating a signal from the sensor (20) when variations in pressure indicate the presence of a defect;
**characterized in that** the nonwoven fibrous material comprises natural fibres, regenerated cellulose fibres, and mixtures thereof.

2. The method according to Claim 1 wherein the detection zone (40) is situated at a nip (13) between a pair of rotating rolls (11, 12) and wherein the nonwoven web (30) is advanced through the nip (13) formed between the pair of rolls (11, 12).

3. The method according to Claim 1 wherein the detection zone (40) is situated on a static surface (22) adjacent to the rotating roll (16) and advancing the nonwoven web (30) between the rotating roll (16) and the static surface (22).

4. The method according to any of Claims 1 to 3 wherein the rotating roll is an idler roll.

5. The method according to any of the previous Claims wherein the sensor (20) is provided on or around the surface of the rotating roll.

6. The method according to any of the previous Claims wherein the sensor (20) is a tactile sensor which is configured to measure pressure up to about 3500 kPa,

7. The method according to Claim 6 wherein the tactile sensor is configured to measure pressure between about 350 kPa and about 3500 kPa.

8. The method according to any of the previous Claims wherein the nonwoven web (30) further comprises thermoplastic fibres.
